# EUROPÄISCHE PATENTSCHRIFT

(11) **EP 0 838 234 B1**
(45) Veröffentlichungstag und Bekanntmachung des Hinweises auf die Patenterteilung: **24.08.2005**
(21) Anmeldenummer: 97250320.5
(22) Anmeldetag: 28.10.1997
(51) Int. Cl.: A61N 1/365, A61N 1/368

(54) **Implantierbares Stimulationsgerät**
Implantable stimulating device
Stimulateur implantable

(30) Priorität: 28.10.1996 DE 19645291; 18.12.1996 DE 19654494
(43) Veröffentlichungstag der Anmeldung: 29.04.1998
(73) Patentinhaber: BIOTRONIK Mess- und Therapiegeräte GmbH & Co Ingenieurbüro Berlin, 12359 Berlin (DE)
(72) Erfinder: Lang, Volker, 91074 Herzogenaurach (DE); Bolz, Armin, Dr., 91054 Buckenhof (DE)
(74) Vertreter: von Oppen, Joachim F.M.

(56) Entgegenhaltungen:
- EP-A- 0 465 241
- EP-A- 0 634 192
- US-A- 4 865 036
- US-A- 5 174 299
- US-A- 5 183 040

## Beschreibung

Die Erfindung betrifft ein implantierbares Stimulationsgerät gemäß dem Oberbegriff des Anspruchs 1.

Es ist bekannt, zur Behandlung von arrhythmischen Funktionsstörungen des Herzens, wie beispielsweise Kammerflimmern, implantierbare Kardioversionseinrichtungen - auch als Defibrillatoren bezeichnet - zu verwenden, die über eine in der Regel endokardial im Ventrikel angeordnete Elektrode spontane Herzsignale erfassen und bei einer Detektion von Herzkammerflimmern oder -flattern einen energiereichen Stromimpuls - im folgenden als Defibrillationsimpuls bezeichnet - an das Herz abgeben, der aufgrund seiner Stärke zu einer Erregung und Depolarisation nahezu sämtlicher Herzbereiche führt, so daß der Arhythmiezustand beendet wird und der normale Sinusrhythmus wieder die Kontrolle über den Herzschlag übernehmen kann.

Eine derartige Erkennung eines arrhythmischen Herzzustands durch Auswertung der intrakardial abgenommenen spontanen Herzsignale unterliegt jedoch vielfältigen Störmöglichkeiten. So kann die Amplitude der spontanen Herzsignale zeitlichen Schwankungen beispielsweise im Tag-Nacht-Rhythmus unterliegen, die eine zuverlässige Detektion von arrhythmischen Herzzuständen erschweren. Auch ist es möglich, daß elektromagnetische Störsignale fälschlicherweise als intrakardiale EKG-Signale erkannt werden. Darüber hinaus besteht die Gefahr, daß spontane atriale Herzsignale elektrisch auf den Ventrikel übersprechen, so daß ein relativ harmloses Vorhofflimmern fälschlicherweise als Kammerflimmern erkannt wird und einen Defibrillationsimpuls auslöst, obwohl kein kritischer Zustand des Patienten vorliegt. Bei den bekannten Defibrillatoren ist die Zuverlässigkeit der Detektion von arrhythmischen Herzzuständen also unbefriedigend.

Es sind weiterhin Herzschrittmacher zur Behandlung einer gestörten Reizüberleitung vom Atrium zum Ventrikel bekannt. Derartige Herzschrittmacher erfassen in der Regel über eine endokardial im Atrium angeordnete Elektrode spontane atrielle Aktionen und geben jeweils um eine vorgegebene AV-Überleitungszeit verzögert nach jeder spontanen atriellen Aktion einen Stimulationsimpuls an den Ventrikel ab. Die Verzögerung der ventrikulären Stimulation nach jeder atriellen Aktion ist erforderlich, um eine optimale Kammerfüllung vor der Kontraktion als Voraussetzung für eine gute Pumpleistung des Herzens zu ermöglichen, wobei der optimale Wert der AV-Überleitungszeit individuell für den jeweiligen Herzschrittmacherträger ist und darüber hinaus auch zeitlichen Schwankungen unterliegen kann. Bei den vorbekannten Herzschrittmachern dieser Art besteht also das Problem, daß die AV-Überleitungszeit meist nicht optimal eingestellt ist, was zu einer Verringerung der Pumpleistung des Herzens führt.

Aus dem US-Patent 5,174,299 ist ein Schrittmacher bekannt, der an eine Elektrodenleitung ein Thermoelement zur Erfassung des Blutflusses in der Vena Cava Superior aufweist.

Aus dem US-Patent 5,183,040 ist ein Schrittmacher bekannt, der in einer Elektrodenleitung einen piezoelektrischen Transducer aufweist, um Blutströmungen in der absteigenden Aorta auf Basis des Dopplereffektes zu erfassen.

Der Erfindung liegt somit die Aufgabe zugrunde, ein implantierbares Stimulationsgerät zu schaffen, bei dem die Steuerung der Impulsabgabe zur Beendigung arrhythmischer Funktionsstörungen besser an die Bedürfnisse des Patienten angepaßt ist.

Die Erfindung wird, ausgehend von einem implantierbaren Stimulationsgerät gemäß dem Oberbegriff des Anspruchs 1, durch die kennzeichnenden Merkmale des Anspruchs 1 gelöst.

Die Erfindung schließt die technische Lehre ein, die Impulsabgabe bei einem gattungsgemäßen implantierbaren Stimulationsgerät in Abhängigkeit von der Pumpleistung des Herzens zu steuern, wobei die Pumpleistung des Herzens durch einen hämodynamischen Sensor erfaßt wird, der in oder an einem Blutgefäß den Bludurchfluß erfaßt. Hierbei hat es sich als besonders vorteilhaft erwiesen, den hämodynamischen Sensor in der Vena Cava oder der Arteria Pulmonalis anzuordnen.

Der Begriff Stimulationsgerät umfaßt im folgenden insbesondere Kardioverter und Defibrillatoren, aber auch moderne multifunktionelle Herzschrittmacher, wobei sich das erfindungsgemäße Prinzip bei der Therapierung von bestimmten Tachykardiezuständen als auch von Herkammerflimmern und ggfs. Herzstillstand einsetzen läßt.

Der hämodynamische Sensor kann die Fließgeschwindigkeit des Blutes quantifizieren.

Gemäß der Erfindung erfaßt der hämodynamische Sensor die Impedanz des Blutes, wobei von der überraschenden Erkenntnis ausgegangen wird, das die Impedanz des Blutes unter anderem von der Fließgeschwindigkeit abhängt. Der hämodynamische Sensor weist deshalb in dieser Variante der Erfindung zwei Meßelektroden auf, die in dem Blutgefäß angeordnet sind und somit eine Impedanzmessung des Blutes ermöglichen. In der bevorzugten Ausführungsform dieser Variante sind die beiden Meßelektroden mit einem in das Stimulationsgerät integrierten Signalgenerator verbunden, der zur Impedanzmessung ein elektrisches Testsignal erzeugt, das auf die beiden Meßelektroden gegeben wird. Hierbei wird von einem ebenfalls in das Stimulationsgerät integrierten Strommeßgerät der Stromfluß über die beiden Meßelektroden gemessen, was anschließend die Berechnung der Impedanz des Blutes ermöglicht, die die Fließgeschwindigkeit des Blutes und damit die Pumpleistung des Herzens widerspiegelt.

In der bevorzugten Ausführungsform dieser Variante sind die beiden Meßelektroden in den Katheter integiert, der zur Plazierung der Stimulationselektrode in das Herz eingeführt wird. Durch die Integration des hämodynamischen Sensors in den Katheter läßt sich das erfindungsgemäße Stimulationsgerät vorteilhaft ohne zusätzlichen Aufwand implantieren, da der Katheter zur Plazierung der Stimulationselektrode ohnehin erforderlich ist. Die beiden Meßelektroden befinden sich hierbei vorzugsweise in der Wandung des Katheters in axialer Richtung zueinander beabstandet an einer Position, die im implantierten Zustand außerhalb des Herzens liegt.

Die beiden Meßelektroden können in dem Blutgefäß in Flußrichtung hintereinander oder quer zur Flußrichtung nebeneinander angeordnet werden, um die Anisotropie der Impedanz des Blutes berücksichtigen zu können, die daher rührt, daß sich die Erythrozyten in einer Blutströmung mitunter in einer Vorzugsrichtung ausrichten.

In der bevorzugten Variante der Erfindung ist die zur Steuerung der Impulsabgabe dienende Steuereinheit eingangsseitig zur Erfassung spontaner Herzsignale und zur Steuerung der Impulsabgabe in Abhängigkeit von dem gemessenen Blutfluß einerseits und den spontanen Herzsignalen andererseits eingangsseitig mit der Stimulationselektrode oder einer weiteren, vorzugsweise endokardial angeordneten Elektrode verbunden. Die Steuerung der Impulsabgabe erfolgt hierbei also nicht nur in Abhängigkeit von dem gemessenen Blutfluß, sondern auch in Abhängigkeit von den spontanen Herzsignalen und läßt sich somit besser an die Bedürfnisse des Patienten anpassen, da der körperliche Zustand des Patienten von der Steuereinheit aufgrund der vielfältigeren diagnostischen Informationen besser erkannt werden kann. Darüber hinaus lassen sich hierdurch solche Störsignale unterdrücken, die sich nur auf eine einzige Eingangsgröße auswirken. So ist es beispielsweise denkbar, daß die gemessenen spontanen Herzsignale durch elektromagnetische Störsignale verfälscht werden, während die gemessene Pumpleistung weitgehend unverfälscht ist. Durch die Auswertung verschiedener Signale bietet sich also die Möglichkeit einer gegenseitigen Plausibilitätsüberprüfung zwischen den verschiedenen Signalen, um Störungen zu unterdrücken.

Zur Auswertung der spontanen Herzsignale weist die Steuereinheit in einer Variante der Erfindung eine Signalanalyseeinheit auf, die eingangsseitig mit der zur Detektion der spontanen Herzsignale dienenden Elektrode verbunden ist und anhand der gemessenen spontanen Herzsignale beurteilt, ob eine Herzfunktionsstörung vorliegt. Dies ist insbesondere bei einer Realisierung der Erfindung in einem Defibrillator vorteilhaft, wobei die Signalanalyseeinheit eine Detektion von Tachykardien oder arrhythmischen Herzfunktionsstörungen, wie Kammerflimmern, ermöglicht und bei einer Erkennung einer derartigen Herzfunktionsstörung ein entsprechendes Steuersignal erzeugt. Die Abgabe von Defibrillationsimpulsen erfolgt hierbei jedoch nicht nur in Abhängigkeit von den spontanen Herzsignalen, sondern wird auch von der Herzpumpleistung beeinflußt, die von dem hämodynamischen Sensor gemessen wird. Hierdurch ist es - wie bereits vorstehend erwähnt - möglich, solche Störsignale auszuscheiden, die sich lediglich auf eine der beiden Eingangssignale auswirken. Dies bedeutet, daß ein Defibrillationsimpuls nur dann abgegeben wird, wenn die spontanen Herzsignale ein Kammerflimmern anzeigen und die Herzpumpleistung unter einen vorgegebenen Grenzwert abgefallen ist, was bei einem Kammerflimmern grundsätzlich der Fall ist. Zu einer derartigen Verknüpfung der Eingangssignale eignet sich hierbei wie auch bei der Realisierung der Erfindung in anderen Stimulationsgeräten vorzugsweise eine Logikeinheit, die auch die Einbeziehung weiterer diagnostischer Größen ermöglicht, wie beispielsweise der von einem Aktivitätssensor erfaßten körperlichen Aktivität oder dem Atemminutenvolumen, das durch Messung der intrakardialen Impedanz ermittelt werden kann.

In einer vorteilhaften Variante der Erfindung ist weiterhin vorgesehen, den hämodynamischen Sensor nicht permanent zu betreiben, um den Energieverbrauch beim Betrieb des Stimulationsgerätes zu senken, was insbesondere dann vorteilhaft ist, wenn der hämodynamische Sensor - wie vorstehend beschrieben - ein beheizbares Widerstandselement aufweist, da der Betrieb des hämodynamischen Sensors in diesem Fall mit einem relativ hohen Energieverbrauch verbunden ist. Die Aktivierung des hämodynamischen Sensors erfolgt in dieser Variante der Erfindung durch ein steuerbares Schaltelement, wobei die Ansteuerung des Schaltelements beispielsweise zeit- oder ferngesteuert über einen Telemetrieempfänger erfolgen. Bei einer Realisierung des erfindungsgemäßen Konzepts in einem Defibrillator ist es jedoch besonders vorteilhaft, den hämodynamischen Sensor zu aktivieren, wenn die Auswertung der spontanen Herzsignale einen arrhythmischen Herzzustand anzeigt. Das Meßergebnis des hämodynamischen Sensors ermöglicht dann eine Plausibilitätsüberprüfung und verhindert somit die Abgabe eines kontraindizierten Defibrillationsimpulses.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: als bevorzugtes Ausführungsbeispiel der Erfindung einen implantierbaren Defibrillator, bei dem ein Defibrillationsimpuls nur bei einem Abfall der Herzpumpleistung abgegeben wird, wobei die Herzpumpleistung durch einen hämodynamischen Sensor gemessen wird,
- Figur 2: als weiteres Ausführungsbeispiel einen Herzschrittmacher, bei dem zur Optimierung der AV-Überleitungszeit die Herzpumpleistung durch einen hämodynamischen Sensor gemessen wird,
- Figur 3a: eine Ausführungsform eines hämodynamischen Sensors für die vorstehend angeführten Ausführungsbeispiele,
- Figur 3b: eine alternative Ausführungsform eines hämodynamischen Sensors,
- Figur 4: den zeitlichen Verlauf des Stromflusses durch den in Figur 3a dargestellten hämodynamischen Sensor sowie
- Figur 5: eine erfindungs gemäße Ausführungsform eines hämodynamischen Sensors, bei dem zur Bestimmung der Herzpumpleistung die Impedanz des Blutes gemessen wird.

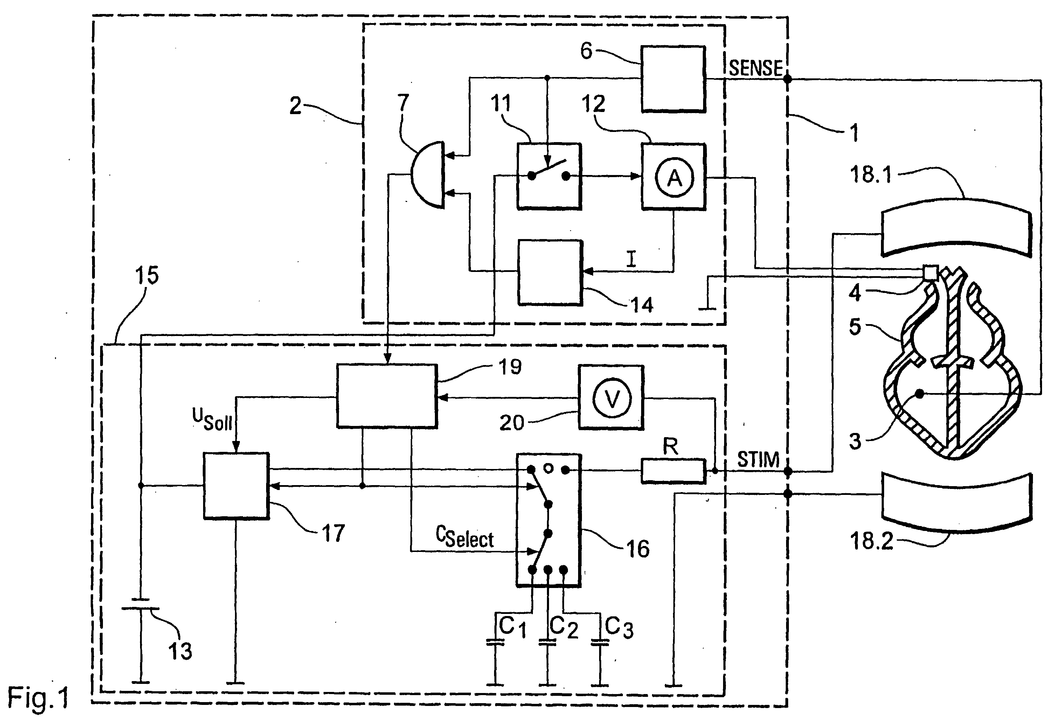

Der in Figur 1 als Blockschaltbild dargestellte implantierbare Defibrillator 1 ermöglicht eine automatische Erkennung und Terminierung eines arrhythmischen Herzzustands durch Abgabe eines energiereichen Defibrillationsimpulses, der aufgrund seiner Stärke zu einer Erregung und Depolarisation nahezu sämtlicher Herzbereiche führt, so daß der Arhythmiezustand beendet wird und der normale Sinusrhythmus wieder die Kontrolle über den Herzschlag übernehmen kann.

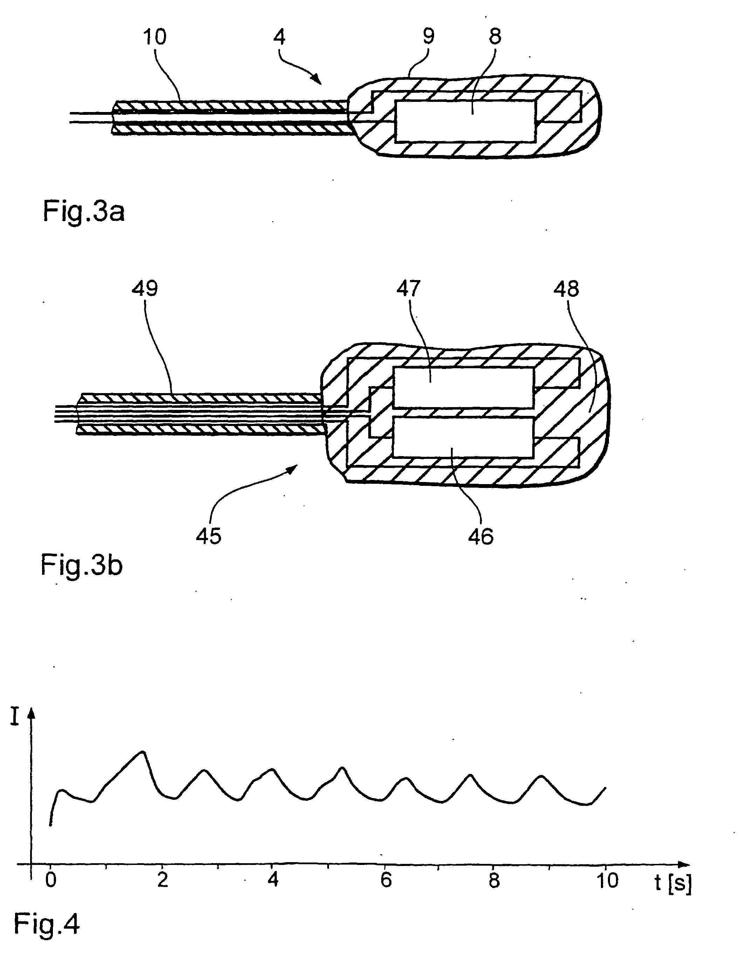

Die Steuerung der Impulsabgabe erfolgt hierbei durch eine Steuereinheit 2, die zur Beurteilung der Herzfunktion eingangsseitig zum einen mit einer endokardial im Ventrikel angeordneten Elektrode 3 und zum anderen mit einem in der Vena Cava angeordneten hämodynamischen Sensor 4 verbunden ist, dessen Aufbau detailliert in Figur 3a dargestellt ist.

Die Elektrode 3 ermöglicht hierbei die Erfassung spontaner Herzsignale und die Erkennung eines arrhythmischen Herzzustands, während der hämodynamische Sensor 4 die Fließgeschwindigkeit des Blutes erfaßt und damit eine Beurteilung der Pumpleistung des Herzens 5 gestattet. Die Steuerung der Impulsabgabe durch unterschiedliche Eingangssignale bietet verschiedene Vorteile. Zum einen erhält die Steuereinheit 2 aufgrund der unterschiedlichen diagnostischen Eingangssignale ein genaueres Bild von der Herzfunktion, so daß die Steuerung der Impulsabgabe besser an die Bedürfnisse des Patienten angepaßt werden kann. Zum anderen ist es aufgrund der unterschiedlichen Eingangssignale möglich, solche Störsignale zu unterdrücken, die sich nur auf eines der Eingangssignale auswirken. Eine derartige gegenseitige Plausibilitätsüberprüfung der beiden Eingangssignale erhöht die Zuverlässigkeit bei der Erkennung von arrhythmischen Herzfunktionsstörungen und vermeidet weitgehend Fehldetektionen und kontraindizierte Defibrillationsimpulse.

Zur Erkennung eines arrhythmischen Herzzustands weist die Steuereinheit 2 eine Signalanalyseeinheit 6 auf, die eingangsseitig zur Aufnahme der spontanen Herzsignale mit der endokardial angeordneten Elektrode 3 verbunden ist. Bei einer Detektion eines arrhythmischen Herzzustands erzeugt die Signalanalyseeinheit 6 ein entsprechendes Steuersignal mit einem logischen HIGH-Pegel, das zur weiteren Auswertung dem UND-Gatter 7 zugeführt wird.

Darüber hinaus führt die Erkennung eines arrhythmischen Herzzustands durch die Signalanalyseeinheit 6 auch zur Aktivierung des hämodynamischen Sensors 4, der daraufhin die Pumpleistung des Herzens 5 ermittelt. Der hämodynamische Sensor 4 ist also im normalen Betrieb des Defibrillators 1 zur Energieeinsparung abgeschaltet und wird nur bei einer Erkennung eines arrhythmischen Herzzustands durch die Signalanalyseeinheit 6 eingeschaltet, um anhand der gemessenen Herzpumpleistung eine Plausibilitätsüberprüfung durchzuführen und eine kontraindizierte Abgabe eines Defibrillationsimpulses zu verhindern.

Der hämodynamische Sensor 4, besteht - wie auch aus Figur 3a ersichtlich - im wesentlichen aus einem Widerstandselement 8 und ist in der Vena Cava angeordnet. Um einen elektrischen Kontakt mit dem vorbeiströmenden Blut oder der Gefäßwand zu verhindern, ist das Widerstandselement 8 mit einer elektrisch isolierenden, aber thermisch gut leitfähigen Masse 9 vergossen, durch die die Zuleitungen 10 hindurchgeführt sind.

Zur Aktivierung des hämodynamischen Sensors 4 wird dieser über das steuerbare Schaltelement 11 und die Strommeßeinrichtung 12 mit der Batterie 13 des Defibrillators 1 verbunden, so daß das Widerstandselement 8 von einem elektrischen Strom durchflossen wird. Aufgrund der ohmschen Verluste im Inneren des Widerstandselements 8 steigt dessen Temperatur dann solange an, bis ein Gleichgewicht erreicht ist zwischen der zugeführten elektrischen Leistung einerseits und der durch Wärmeleitung und Konvektion von dem vorbeiströmenden Blut abgeführten thermischen Leistung andererseits. Da die durch Konvektion abgeführte Leistung mit der Fließgeschwindigkeit des vorbeiströmenden Blutes zunimmt, ermöglicht eine Messung der Gleichgewichtstemperatur eine Bestimmung der Fließgeschwindigkeit. So wird bei einer geringen Fließgeschwindigkeit nur wenig Wärme durch Konvektion abgeführt, so daß sich erst ein relativ hoher Temperaturgradient am Widerstandselement 8 aufbauen muß, um die zugeführte Wärme durch Wärmeleitung abführen zu können, was im Ergebnis zu einer relativ hohen Temperatur des Widerstandselements 8 führt. Mit zunehmender Fließgeschwindigkeit des vorbeiströmenden Blutes wird dann entsprechend mehr Wärme durch Konvektion abgeführt, so daß der Temperaturgradient an dem Widerstandselement 8 abnehmen kann, was zu einer Temperaturabnahme des Widerstandselements 8 führt. Die Fließgeschwindigkeit des Blutes in der Vena Cava und damit die Pumpleistung des Herzens läßt sich also durch eine Messung des elektrischen Stroms durch das Widerstandselement 8 ermitteln, da der ohmsche Widerstand des Widerstandselements 8 temperaturabhängig ist. Figur 4 zeigt beispielhaft den pulsierenden Verlauf des elektrischen Stroms durch das Widerstandselements 8 für eine normale Herzrate. Deutlich sind hierbei die Schwankungen des Stromflusses zu erkennen, die den Herzrhythmus widerspiegeln. Das Ausgangssignal der Strommeßeinrichtung 12 wird deshalb einer Signalanalyseeinheit 14 zugeführt, die daraus die Pumpleistung des Herzens 5 berechnet und diesen Wert mit einem vorgegebenen Grenzwert vergleicht. Beim Absinken der Pumpleistung unter den vorgegebenen Grenzwert nimmt die Signalanalyseeinheit 14 als Ursache einen arrhythmischen Herzzustand an und erzeugt deshalb ausgangsseitig ein entsprechendes Steuersignal mit einem HIGH-Pegel, das nachfolgend zur weiteren Verarbeitung dem UND-Gatter 7 zugeführt wird.

Das UND-Gatter 7 dient zur Triggerung des Impulsgenerators 15 zur Abgabe eines Defibrillationsimpulses im Falle eines arrhythmischen Herzzustands, wobei aufgrund der UND-Verknüpfung nur dann ein Defibrillationsimpuls abgegeben wird, wenn sowohl die Auswertung der spontanen Herzsignale als auch die Auswertung der Pumpleistung des Herzens 5 ein Kammerflimmern oder -flattern anzeigt. Durch diese Plausibilitätsüberprüfung wird eine kontraindizierte Abgabe eines Defibrillationsimpulses bei einer gestörten Signalerfassung aus dem Herzen 5 verhindert. Insbesondere wird hierdurch verhindert, daß beim elektrischen Übersprechen eines relativ harmlosen Vorhofflimmerns vom Atrium auf den Ventrikel fälschlicherweise ein Kammerflimmern detektiert und ein Defibrillationsimpuls abgegeben wird, der in diesem Fall kontraindiziert wäre.

Die Erzeugung der Defibrillationsimpulse erfolgt durch den Impulsgenerator 15, der von dem UND-Gatter 7 der Steuereinheit 2 angesteuert wird und beim Vorliegen eines arrhythmischen Herzzustands zunächst einen der Pufferkondensatoren C₁, C₂, C₃ während einer Ladephase durch die Schaltmittel 16 mit der Spannungsregelschaltung 17 verbindet, wodurch der Pufferkondensator aufgeladen wird. Die Spannungsregelschaltung 17 ist eingangsseitig zur Energieversorgung mit der Batterie 13 verbunden und stellt eine Ausgangsspannung bereit, deren Höhe zwischen 560 V und 800 V quasi stufenlos eingestellt werden kann. Nach der Ladephase mit einer vorgegebenen Zeitdauer wird der ausgewählte Pufferkondensator C₁, C₂ oder C₃ dann durch die Schaltmittel 16 von der Spannungsregelschaltung 17 getrennt und über den Ausgangswiderstand R mit der Stimulationselektrode 18.1 verbunden, so daß sich der Pufferkondensator C₁, C₂ bzw. C₃ über das Herz 5 und die Masseelektrode 18.2 entlädt und einen Defibrillationsimpuls abgibt. Wegen der relativ großen Impulsstärke ist die Stimulationselektrode 18.1 wie auch die Masseelektrode 18.2 - im Gegensatz zu der Detektionselektrode 3 - großflächig ausgeführt, um Gewebeverbrennungen oder -schädigungen bei der Abgabe der energiereichen Defibrillationsimpulse zu verhindern.

Die zeitliche Steuerung der Schaltmittel 16 zur Aufladung des Pufferkondensators bzw. zur Abgabe des Defibrillationsimpulses erfolgt hierbei durch die Steuereinheit 19. Darüber hinaus ermöglicht die Steuereinheit 19 auch eine Anpassung der Defibrillationsenergie an die individuelle Defibrillationsschwelle des Patienten, indem entweder die von der Spannungsregelschaltung 17 bereitgestellte Ladespannung oder die Kapazität des Pufferkondensators verändert wird. Die Steuereinheit 19 ist deshalb zur Einstellung der Ladespannung ausgangsseitig mit der Spannungsregelschaltung 17 und zur Auswahl eines der Pufferkondensatoren C₁, C₂, C₃ mit den Schaltmitteln 16 verbunden.

Anschließend wird der Pufferkondensator durch die Schaltmittel 16 wieder von der Stimulationselektrode 18.1 getrennt, wodurch der Defibrillationsimpuls beendet wird.

Einerseits ist es zur erfolgreichen Defibrillation wichtig, daß der Pufferkondensator C₁, C₂ bzw. C₃ zur Beendigung eines Defibrillationsimpulses rechtzeitig von der Stimulationselektrode 18.1 getrennt wird, bevor die Kondensatorspannung aufgrund der Entladung unter den Rheobasewert fällt, da eine weitere Entladung nicht defibrillierend wirkt, sondern allenfalls ein Refibrillieren einleiten kann.

Andererseits ist es wünschenswert, den Pufferkondensator C₁, C₂ bzw. C₃ möglichst vollständig zu entladen, da die nach einem Defibrillationsimpuls auf dem Pufferkondensator C₁, C₂ bzw. C₃ verbleibende Restladung nicht oder nur unvollständig genutzt werden kann und somit den Wirkungsgrad des Defibrillators 1 herabsetzt, der als Verhältnis von gespeicherter Energie zu abgegebener Energie definiert ist.

Der Defibrillator 1 mißt deshalb während der Abgabe eines Defibrillationsimpulses laufend die Stimulationsspannung und beendet den Defibrillationsimpuls beim Unterschreiten der Rheobasespannung. Hierzu weist der Defibrillator 1 eine Spannungsmeßeinrichtung 20 auf, die eingangsseitig mit dem Anschluß für die Stimulationselektrode 18.1 und ausgangsseitig mit der Steuereinheit 19 verbunden ist, um die Defibrillationsspännung messen und den Defibrillationsimpuls gegebenenfalls beenden zu können. Beim Erreichen der Rheobasespannung erzeugt die Steuereinheit 19 dann ein Steuersignal für die Schaltmittel 16, die daraufhin den Pufferkondensator C₁, C₂ bzw. C₃ von der Stimulationselektrode 18.1 trennen und damit den Defibrillationsimpuls beenden. Zum einen wird auf diese Weise vorteilhaft verhindert, daß ein Defibrillationsimpuls beim Absinken der Defibrillationsspannung unter die Rheobasespannung refibrillierend wirkt. Zum anderen kann durch die laufende Messung der Defibrillationsspannung bei der Entladung des Pufferkondensators C₁, C₂ bzw. C₃ auf einen Sicherheitsabstand zwischen Defibrillationsspannung und Rheobasespannung verzichtet werden, so daß der Pufferkondensator C₁, C₂ bzw. C₃ bis zur Rheobasespannung entladen werden kann, wodurch die Batterielebensdauer erhöht wird.

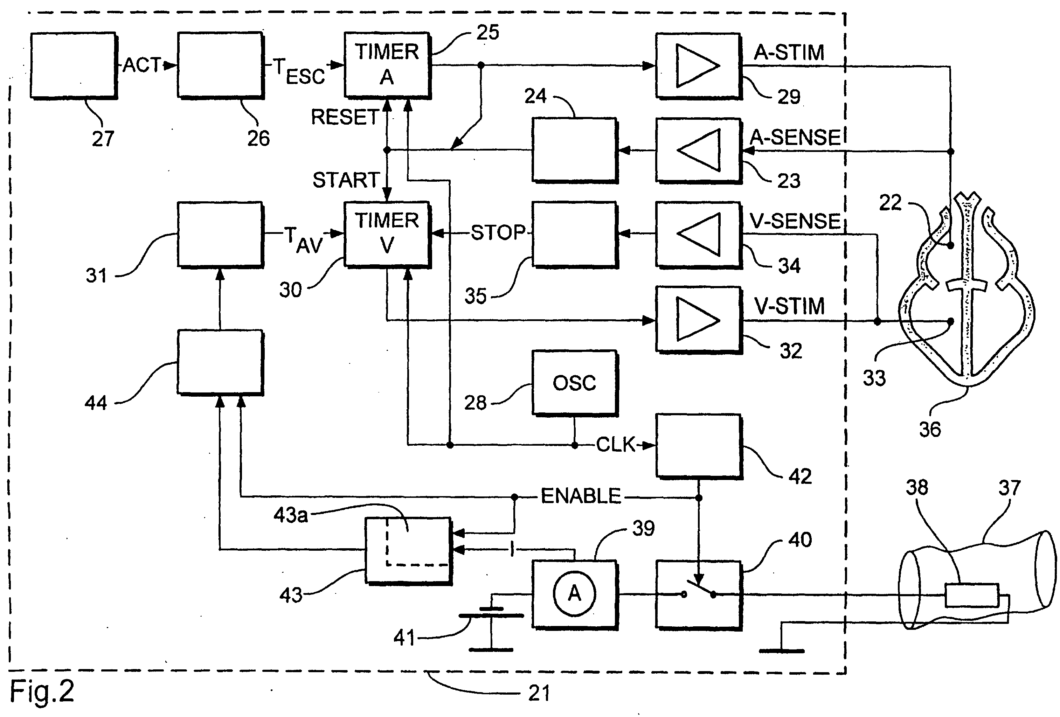

Figur 2 zeigt als weiteres Ausführungsbeispiel der Erfindung einen ratenadaptiven Zweikammer-Herzschrittmacher 21 zur Behandlung einer gestörten Reizüberleitung vom Atrium zum Ventrikel. Hierzu erfaßt der dargestellte Schrittmacher 21 über eine endokardial (im Atrium) plazierte Elektrode 22 spontane Herzsignale, die zunächst einem Eingangsverstärker 23 und anschließend einer Signalanalyseeinheit 24 zugeführt werden, um eine spontane atrielle Aktion zu detektieren, die in den erfaßten Herzsignalen als P-Welle auftritt. Bei der Detektion einer derartigen P-Welle erzeugt die Signalanalyseeinheit 24 ein Steuersignal, das den Zähler 25 initialisiert, wobei der Startwert des Zählers 25 von der Recheneinheit 26 aus dem Aktivitätssignal ACT berechnet wird, das von dem piezoelektrischen Beschleunigungssensor 27 erzeugt wird und die körperliche Aktivität des Herzschrittmacherträgers widerspiegelt. Bei jedem Taktimpuls des Oszillators 28 verringert der Zähler 25 dann seinen Zählwert bis auf Null, woraufhin der Zähler 25 ein Steuersignal an den Impulsgenerator 29 abgibt, der daraufhin einen Stimulationsimpuls erzeugt und über die Elektrode 22 dem Atrium zuführt.

Darüber hinaus ermöglicht der dargestellte Herzschrittmacher 21 eine Stimulation des Ventrikels, was insbesondere bei einer gestörten Reizüberleitung vom Atrium zum Ventrikel wichtig ist. Die Stimulation im Ventrikel erfolgt hierbei jeweils um eine vorgegebene AV-Überleitungszeit verzögert nach der vorangegangenen spontanen oder stimulierten atriellen Aktion, damit sich der Ventrikel vor der Kontraktion möglichst vollständig mit Blut füllen kann, um bei einer vorgegebenen Herzrate ein möglichst großes Herzminutenvolumen zu erreichen.

Der Zähler 30 für die Zeitsteuerung des Ventrikels ist deshalb eingangsseitig zum einen mit der Signalanalyseeinheit 24 und zum anderen mit dem Zähler 25 für die atriale Zeitsteuerung verbunden, um sowohl bei einer spontanen als auch bei einer stimulierten atriellen Aktion eine AV-Überleitungszeit zu starten, wobei der Startwert für den Zähler 30 in dem Speicherelement 31 abgelegt ist. Bei jedem Taktimpuls des Oszillators 28 dekrementiert der Zähler 30 dann den Zählwert bis auf Null, woraufhin ein Steuersignal an den Impulsgenerator 32 abgegeben wird, der daraufhin einen Stimulationsimpuls erzeugt und über die Elektrode 33 an den Ventrikel abgibt. Die Stimulation des Ventrikels erfolgt jedoch nicht starr nach jeder atriellen atriellen Aktion, sondern kann durch eine während der AV-Überleitungszeit auftretende spontane ventrikuläre Aktion inhibiert werden. Die im Ventrikel angeordnete Elektrode 33 ist hierzu mit einem Eingangsverstärker 34 und einer nachgeschalteten Signalanalyseeinheit 35 verbunden, um eine spontane Ventrikelaktion zu detektieren, die in den erfaßten Herzsignalen als QRS-Komplex auftritt. Bei einer Detektion eines derartigen QRS-Komplexes erzeugt die Signalanalyseeinheit 35 ein Inhibierungssignal, das dem Zähler 30 zugeführt wird, der daraufhin seinen Zählvorgang abbricht und somit auch keinen Stimulationsimpuls anregt.

Der Schrittmacher 21 ermöglicht eine automatische Einstellung der AV-Überleitungszeit.

Hierzu mißt der Herzschrittmacher 21 über einen hämodynamischen Sensor die Fließgeschwindigkeit des Blutes in der Vena Cava 37, um die Pumpleistung des Herzens 36 beurteilen zu können. Der hämodynamische Sensor besteht im wesentlichen aus einem in der Vena Cava angeordneten Widerstandselement 38 mit einem temperaturabhängigen elektrischen Widerstand, das während eines Meßvorgangs über die Strommeßeinrichtung 39 und das steuerbare Schaltelement 40 mit der Batterie 41 verbunden wird, wodurch sich das Widerstandselement 38 aufgrund der ohmschen Verluste in seinem Inneren erhitzt.

Da die Messung der Fließgeschwindigkeit durch das Widerstandselement 38 mit einem zusätzlichen Stromverbrauch verbunden ist, erfolgt die Messung nicht permanent, sondern jeweils zu programmierten Zeitpunkten, Z.B. mit einem Zeitabstand von einigen Stunden. Hierzu weist der dargestellte Herzschrittmacher 21 einen weiteren Zähler 42 auf, der ebenfalls von dem Oszillator 28 angesteuert wird und jeweils beim Erreichen eines bestimmten Zählerstandes ein Steuersignal erzeugt, das dem steuerbaren Schaltelement 40 zugeführt wird, woraufhin dieses das Widerstandselement 38 über die Strommeßeinrichtung 39 mit der Batterie 41 verbindet.

Die Temperatur des Widerstandselements 38 nimmt dann solange zu, bis sich ein Gleichgewicht einstellt zwischen der zugeführten und im Widerstandselement 38 vollständig in Wärme umgewandelten Leistung einerseits und der durch Wärmeleitung und Konvektion von dem vorbeiströmenden Blut abgeführten thermischen Leistung andererseits. Da die durch Konvektion abgeführte Leistung mit der Fließgeschwindigkeit des Blutes zunimmt, besteht eine Abhängigkeit zwischen der Temperatur des Widerstandselements 38 und der Fließgeschwindigkeit des Blutes. So wird bei geringen Fließgeschwindigkeiten nur wenig Wärme durch Konvektion abgeführt, so daß zur Abführung der zugeführten elektrischen Energie ein relativ großer Temperaturgradient am Widerstandselement 38 und damit eine entsprechend hohe Temperatur erforderlich ist. Bei größeren Fließgeschwindigkeiten wird dagegen mehr Wärme durch Konvektion abgeführt, so daß der Anteil der Wärmeleitung geringer ausfällt und somit auch ein geringerer Temperaturgradient am Widerstandselement 38 ausreicht, um die Leistungsbilanz des Widerstandselements 38 im Gleichgewicht zu halten. Die Temperatur des Widerstandselements 38 spiegelt also die Fließgeschwindigkeit des Blutes wider und läßt sich aufgrund der temperaturabhängigen elektrischen Leitfähigkeit des Widerstandselements 38 in einfacher Weise aus dem Stromfluß durch das Widerstandselement 38 berechnen. Das Ausgangssignal der Strommeßeinrichtung 39 wird deshalb als Maß für die Fließgeschwindigkeit der Recheneinheit 43 zugeführt, die daraus das Herzzeitvolumen (HZV) berechnet, das die Pumpleistung wiedergibt. Die Recheneinheit 43 umfaßt eine Kompensationsstufe 43a zur Kompensation langfristiger Meßwertschwankungen aufgrund von Schwankungen des Basistemperatur. Diese wird zu Beginn jedes Meßvorganges, slso vor Verbindung des Widerstandselements mit der Batterie 41, durch das steuerbare Schaltelement 40 aktiviert und stellt intern einen Korrekturwert bereit, der in das Berechnungsergebnis der Einheit 43 einfließt.

Die Optimierung der AV-Überleitungszeit erfolgt dann durch eine Steuerstufe 44, die eingangsseitig mit der Recheneinheit 43 verbunden ist und das Herzminutenvolumen als Optimierungskriterium aufnimmt. Ausgangsseitig ist die Steuerstufe 44 mit dem Speicherelement 31 verbunden, das jeweils den aktuellen Wert der AV-Überleitungszeit aufnimmt und zwischen den einzelnen Meßvorgängen des hämodynamischen Sensors festhält.

Figur 3b zeigt eine alternative Ausführungsform eines hämodynamischen Sensors 45, die sich von dem bereits vorstehend beschriebenen und in Figur 3a dargestellten Sensor 4 im wesentlichen dadurch unterscheidet, daß die Aufheizung des Widerstandselements 46 nicht durch die in seinem Inneren auftretenden ohmschen Verluste, sondern durch ein separates Heizelement erfolgt. Dieses Heizelement besteht ebenfalls aus einem Widerstandselement 47, daß zur Aufheizung mit einer Strom- bzw. Spannungsquelle verbunden wird und sich somit durch die in seinem Inneren auftretenden ohmschen Verluste aufheizt. Die Aufheizung des zur Messung dienenden Widerstandselements 46 erfolgt dann durch Wärmeleitung zwischen den beiden Widerstandselementen 46, 44. Zur Verhinderung eines elektrischen Kontakts mit dem vorbeiströmenden Blut sind die beiden Widerstandselemente 46, 47 mit einer elektrisch isolierenden, aber thermisch gut leitfähigen Masse 48 vergossen.

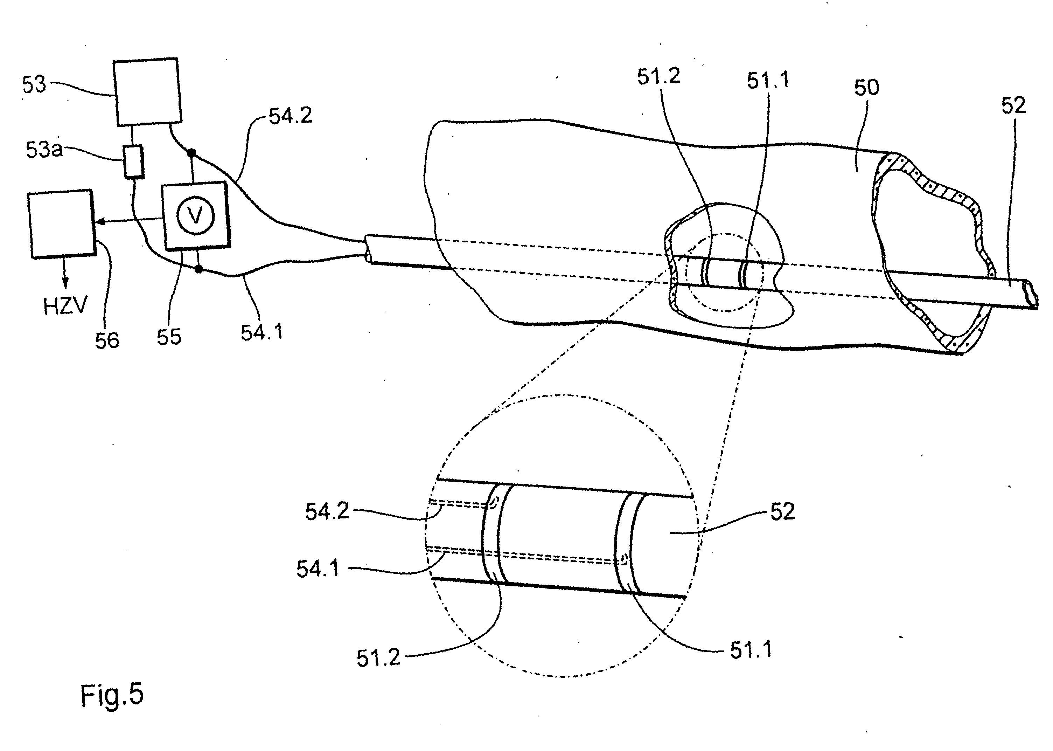

Figur 5 zeigt eine weitere Ausführungsform eines hämodynamischen Sensors. Bei diesem wird die Impedanz des Blutes in einem Blutgefäß 50 gemessen, die sich im Bereich unterhalb von etwa 10 kHz (in dem die Erythrozyten nach Feststellung der Erfinder faktisch als Isolatoren betrachet werden können) im Ergebnis der in Abhängigkeit von der Fließgeschwindigkiet mehr oder weniger uniformen Orientierung der Erythrozyten fließgeschwindigkeitsabhängig ist.

Die Impedanzmessung erfolgt hierbei durch zwei Meßelektroden 51.1, 51.2, die in der Wandung eines Katheters 52 angeordnet sind, dessen distales Ende im Ventrikel fixiert ist und die Stimulationselektrode trägt. Durch die Integration des hämodynamischen Sensors in den ohnehin erforderlichen Katheter 52 ist die Implantation der Stimulationsanotrdnung vorteilhaft ohne zusätzlichen chirurgischen Aufwand möglich. Darüber hinaus wird hierdurch einer Dislokation der Meßelektroden 51.1, 51.2 entgegengwirkt und eine räumliche Fixierung der beiden Meßelektroden 51.1, 51.2 relativ zueinander sichergestellt. Die beiden Meßelektroden 51.1, 51.2 sind hierbei in einem Bereich des Katheters 52 angeordnet, der im implantierten Zustand außerhalb des Herzens in einem Blutgefäßabschnitt mit im wesentlichen laminarer Strömung liegt, damit die Bestimmung der Fließgeschwindigkeit nicht durch Strömungsturbulenzen gestört wird.

Zur Messung der Impedanz erzeugt ein in das Stimulationsgerät integrierter, im Bereich zwischen 0,1 Hz und 10 kHz arbeitender und eine sinusförmige Meß- bzw. Abtastspannung von ± 1 V liefernder Signalgenerator 53 ein elektrisches Testsignal, das über zwei im Inneren des Katheters 52 verlaufende elektrische Leitungen 54.1, 54.2 und einen Serienwiderstand 53a von 10 kΩ zur Meßstromreduizierung den beiden Meßelektroden 51.1, 51.2 zugeführt wird. Ein zwischen die beiden Meßleitungen 54.1, 54.2 geschaltetes Effektivspannungs-Meßgerät 55 mißt den Effektivwert des Spannungsabfalls über den Meßelektroden 51.1, 51.2. Das auf diese Weise gewonnene Signal wird dann einer Auswertungseinheit 56 zugeführt, die hieraus die Impedanz des Blutes berechnet und in Abhängigkeit davon - auf der Basis einer vorgespeicherter Kennlinien den Blutfluß in dem Blutgefäß 50 ermittelt, der das Herzzeitvolumen (HZV) widerspiegelt und eine Optimierung des Stimulationsverhaltens ermöglicht.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht.

## Patentansprüche

1. Implantierbares Stimulationsgerät (1, 21) zur Behandlung arrhythmischer Funktionsstörungen des Herzens, mit einer im Herzen (5, 36) oder in dessen Umgebung angeordneten Stimulationselektrode (18.1, 22, 33) zur Abgabe von Stimulationsimpulsen an das Herz,
einem ausgangsseitig mit der Stimulationselektrode verbundenen Impulsgenerator (15, 29, 32) zur Erzeugung der Stimulationsimpulse sowie
einer ausgangsseitig mit dem Impulsgenerator (15, 29, 32) verbundenen Steuereinheit (2) zur Steuerung der Impulsabgabe durch den Impulsgenerator, die zur Steuerung der Impulsabgabe in Abhängigkeit von der Herzpumpleistung eingangsseitig mit einem hämodynamischen Sensor (4, 38 bis 41) verbunden ist,
wobei der Sensor zur Messung der Fließgeschwindigkeit des Blutes in einem Blutgefäß (37, 50) angeordnet ist und ihm zur Erkennung einer arrhythmischen Herzfunktionsstörung anhand der gemessenen Fließgeschwindigkeit des Blutes eine Blutdurchfluss-Signalanalyseeinheit (14) nachgeschaltet ist,
**dadurch gekennzeichnet,**
**dass** der hämodynamische Sensor (50 bis 56) zur Messung der von der Fließgeschwindigkeit abhängigen Impedanz des Blutes zwei zueinander beabstandet angeordnete Messelektroden (51.1, 51.2) aufweist, die außerhalb des Herzens in dem Blutgefäß (50) angeordnet sind,
**dass** zur Erzeugung eines Testsignals für die Impedanzmessung ein eine Wechselspannung im Bereich unterhalb von 10 kHz erzeugender Signalgenerator (53) vorgesehen ist, der ausgangsseitig über einen Serienwiderstand (53a) zur Messstromreduzierung mit den beiden Messelektroden (51.1, 51.2) oder zur Erzeugung eines elektrischen Feldes zwischen den beiden Messelektroden (51.1, 51.2) mit zwei weiteren Elektroden verbunden ist,
**dass** mit den beiden Messelektroden (51.1, 51.2) eine elektrische Messeinrichtung (55) verbunden ist, die ausgangsseitig zur Bestimmung der Fließgeschwindigkeit des Blutes in Abhängigkeit von dem gemessenen elektrischen Signal mit der Blutdurchfluss-Signalanalyseeinheit (14; 56) verbunden ist.

2. Stimulationsgerät (1, 21) nach Anspruch 1, **dadurch gekennzeichnet, daß** die Steuereinheit (2) zur Erfassung spontaner Herzsignale und zur Steuerung der Impulsabgabe in Abhängigkeit von dem gemessenen Blutdurchfluß einerseits und den spontanen Herzsignalen andererseits eingangsseitig mit der Stimulationselektrode (22, 33) oder einer weiteren, zur Erfassung spontaner Herzsignale dienenden Elektrode (3) verbunden ist, welcher zur Erkennung einer Herzfunktionsstörung anhand der gemessenen spontanen Herzsignale eine erste Herzsignalanalyseeinheit (6, 24, 35) nachgeschaltet ist.

3. Stimulationsgerät (1, 21) nach einem der vorhergehenden Ansprüche, **dadurch gekennzeichnet, daß** zur Ein- bzw. Abschaltung des hämodynamischen Sensors (4, 38 bis 41) ein steuerbares Schaltelement (11, 40) vorgesehen ist.

4. Stimulationsgerät (1, 21) nach Anspruch 3, **dadurch gekennzeichnet, daß** ein Steuereingang des steuerbaren Schaltelements (11) zur Einschaltung bei einer Detektion einer Herzfunktionsstörung durch die Herzsignalanalyseeinheit (6) und zur Abschaltung bei einer von der ersten Signalanalyseeinheit (6) ermittelten korrekten Herzfunktion mit dem Ausgang der Herzsignalanalyseeinheit (6) verbunden ist.

5. Stimulationsgerät (1, 21) nach Anspruch 3 oder 4, **dadurch gekennzeichnet, daß** der Steuereingang des steuerbaren Schaltelements (11, 40) zur zeitgesteuerten Ein- bzw. Abschaltung des hämodynamischen Sensors (4, 38 bis 41) mit einem Zeitgeber (28, 42) oder zur ferngesteuerten Ein- bzw. Abschaltung durch ein extrakorporales Kontrollgerät mit einem Telemetrieempfänger verbunden ist.

6. Stimulationsgerät (1, 21) nach Anspruch 5**, dadurch gekennzeichnet, daß** die Steuereinheit (2) zur Auswertung sowohl des gemessenen Blutdurchflusses als auch der Herzsignale eine Logikeinheit (7) aufweist, die eingangsseitig zur Beurteilung der Herzsignale mit der Herzsignalanalyseeinheit (6) und zur Beurteilung der Herzpumpleistung mit der Blutdurchfluß-Signalanalyseeinheit (14) verbunden ist.

7. Stimulationsgerät (1, 21) nach Anspruch 1, **dadurch gekennzeichnet, daß** die beiden Meßelektroden (51.1, 51.2) in dem Blutgefäß (50) zur longitudinalen Impedanzmessung in Flußrichtung hintereinander oder zur transversalen Impedanzmessung quer zur Flußrichtung nebeneinander angeordnet sind.

8. Stimulationsgerät (1, 21) nach einem der vorhergehenden Ansprüche, **dadurch gekennzeichnet, daß** der hämodynamische Sensor (4, 45) in der Vena Cava (37) oder in der Arteria Pulmonalis angeordnet ist.

9. Stimulationsgerät nach einem der Ansprüche 1 bis 8, **dadurch gekennzeichnet, daß** durch den Signalgenerator (53) und den Serienwiderstand (53a) eine Meßspannung im Bereich von 0.3 V bis 3 V, insbesondere von etwa 1 V, und ein Meßstrom im Bereich von 10 µA bis 100 µA eingestellt wird.

10. Stimulationsgerät nach einem der vorhergehenden Ansprüche, **dadurch gekennzeichnet, daß** Mittel (43a) zur Kompensation des Einflusses von temperatur- und/oder medikationsbedingten Blutdurchflußsignaländerungen vorgesehen sind.

## Claims

1. An implantable stimulation device (1, 21) for treating arrhythmic functional disturbances of the heart, comprising
a stimulation electrode (18.1, 22, 33) arranged in the heart (5, 36) or in the vicinity thereof for the delivery of stimulation pulses to the heart,
a pulse generator (15, 29, 32) connected on the output side to the stimulation electrode for generating the stimulation pulses, and
a control unit (2) connected on the output side to the pulse generator (15, 29, 32) for controlling the pulse delivery by the pulse generator, which is connected to a haemodynamic sensor (4, 38 to 41) on the input side for controlling the pulse delivery in dependence on the cardiac pumping capacity,
wherein the sensor for measuring the flow speed of the blood is arranged in a blood vessel (37, 50) and connected downstream thereof for recognising an arrhythmic cardiac functional disturbance on the basis of the measured flow speed of the blood is a blood through-flow signal analysis unit (14),
**characterised in that**
the haemodynamic sensor (50 to 56) for measuring the impedance of the blood, which is dependent on the flow speed, has two mutually spaced measuring electrodes (51.1, 51.2) which are arranged outside the heart in the blood vessel (50),
to produce a test signal for impedance measurement there is provided a signal generator (53) which generates an ac voltage in the range below 10 kHz and which on the output side is connected by way of a series resistor (53a) for measurement current reduction to the two measuring electrodes (51.1, 51.2) or for generating an electrical field between the two measuring electrodes (51.1, 51.2) to two further electrodes, and
connected to the two measuring electrodes (51.1, 51.2) is an electrical measuring device (55) which on the output side for determining the flow speed of the blood in dependence on the measured electrical signal is connected to the blood through-flow signal analysis unit (14; 56).

2. A stimulation device (1, 21) according to claim 1 **characterised in that** for detecting spontaneous cardiac signals and for controlling the pulse delivery in dependence on the measured blood through-flow on the one hand and the spontaneous cardiac signals on the other hand the control unit (2) is connected on the input side to the stimulation electrode (22, 33) or a further electrode (3) which serves for detecting spontaneous cardiac signals and downstream of which is connected a first cardiac signal analysis unit (6, 24, 35) for recognising a cardiac functional disturbance on the basis of the measured spontaneous cardiac signals.

3. A stimulation device (1, 21) according to one of the preceding claims **characterised in that** there is provided a controllable switching element (11, 40) for switching the haemodynamic sensor (4, 38 to 41) on and off.

4. A stimulation device (1, 21) according to claim 3 **characterised in that** a control input of the controllable switching element (11) is connected to the output of the cardiac signal analysis unit (6) for switching on upon detection of a cardiac functional disturbance by the cardiac signal analysis unit (6) and for switching off in the event of a correct cardiac function ascertained by the first signal analysis unit (6).

5. A stimulation device (1, 21) according to claim 3 or claim 4 **characterised in that** the control input of the controllable switching element (11, 40) is connected to a timer (28, 42) for timed switching on and off of the haemodynamic sensor (4, 38 to 41) or to a telemetry receiver for remote-controlled switching on or off by an extracorporeal control device.

6. A stimulation device (1, 21) according to claim 5 **characterised in that** for evaluation both of the measured blood through-flow and also the cardiac signals the control unit (2) has a logic unit (7) which on the input side is connected to the cardiac signal analysis unit (6) for assessing the cardiac signals and to the blood through-flow signal analysis unit (14) for assessing the cardiac pumping capacity.

7. A stimulation device (1, 21) according to claim 1 **characterised in that** the two measuring electrodes (51.1, 51.2) are arranged in succession in the flow direction in the blood vessel (50) for longitudinal impedance measurement or are arranged in mutually juxtaposed relationship transversely with respect to the flow direction for transverse impedance measurement.

8. A stimulation device (1, 21) according to one of the preceding claims **characterised in that** the haemodynamic sensor (4, 45) is arranged in the vena cava (37) or in the pulmonary artery.

9. A stimulation device according to one of claims 1 to 8 **characterised in that** a measuring voltage in the range of 0.3 V to 3 V, in particular about 1 V, and a measuring current in the range of 10 µA to 100 µA is set by the signal generator (53) and the series resistor (53A).

10. A stimulation device according to one of the preceding claims **characterised in that** there are provided means (43a) for compensating for the influence of temperature-induced and/or medication-induced changes in the blood through-flow signal.

## Revendications

1. Appareil de stimulation implantable (1,21) pour le traitement de perturbations du fonctionnement du coeur par arythmie, comportant
une électrode de stimulation (18.1,22,33) disposée dans le coeur (5,36) ou dans son environnement, pour délivrer des impulsions de stimulation au coeur,
un générateur d'impulsions (15,29,32) relié côté sortie à l'électrode de stimulation pour la production des impulsions de stimulation, et
une unité de commande (2) qui est reliée côté sortie au générateur d'impulsions (15,29,32) pour la commande de la délivrance d'impulsions par le générateur d'impulsions et qui est reliée côté entrée, pour la commande de la délivrance d'impulsions et en fonction de la puissance de pompage du coeur, relié, à un capteur hémodynamique (4,38 à 41), et
dans lequel le capteur est disposé pour la mesure de la vitesse de l'écoulement du sang dans un vaisseau sanguin (37,50) et une unité (14) d'analyse du signal de débit sanguin est installée en aval du capteur pour identifier une perturbation de fonctionnement du coeur par arythmie sur la base de la vitesse d'écoulement mesurée du sang,
**caractérisé en ce que**
le capteur hémodynamique (50 à 56) comporte, pour la mesure de l'impédance du sang qui dépend de la vitesse d'écoulement, deux électrodes de mesure (51.1, 51.2) disposées à distance l'une de l'autre et qui sont installées à l'extérieur du coeur dans le vaisseau sanguin (50),
que pour la production d'un signal de test pour la mesure d'impédance, il est prévu un générateur de signaux (53), qui produit une tension alternative dans une gamme inférieure à 10 kHz et qui est relié côté sortie, par l'intermédiaire d'une résistance série (53a) pour la réduction du courant de mesure aux deux électrodes de mesure (51.1,51.2) ou pour la production d'un champ électrique entre les deux électrodes de mesure (51.1, 51.2), à deux autres électrodes,
qu'aux deux électrodes de mesure (51.1,51.2) est relié un dispositif de mesure électrique (55), qui est relié côté sortie à l'unité (14;56) d'analyse du signal de débit sanguin pour la détermination de la vitesse d'écoulement du sang en fonction du signal électrique mesuré.

2. Appareil de stimulation (1,21) selon la revendication 1, **caractérisé en ce que** l'unité de commande (2) servant à détecter des signaux cardiaques spontanés et à commander la délivrance d'impulsions en fonction du débit sanguin mesuré d'une part et des signaux cardiaques spontanés d'autre part est reliée côté entrée à l'électrode de stimulation (22,33) ou à une autre électrode (3) utilisée pour la détection de signaux cardiaques spontanés et à laquelle une première unité (6,24,35) d'analyse de signaux cardiaques est branchée pour l'identification d'une perturbation du fonctionnement du coeur sur la base des signaux cardiaques spontanés mesurés.

3. Appareil de stimulation (1,21) selon l'une des revendications précédentes, **caractérisé en ce que** pour le branchement ou le débranchement du capteur hémodynamique (4,38 à 41), il est prévu un élément de commutation commandable (11,40).

4. Appareil de stimulation (1,21) selon la revendication 3, **caractérisé en ce qu'**une entrée de commande de l'élément de commutation commandable (11) est reliée à la sortie de l'unité (6) d'analyse du signal cardiaque pour le branchement lors d'une détection d'une fonction de perturbation du coeur et pour le débranchement dans le cas d'une fonction cardiaque correcte déterminée par la première unité (6) d'analyse du signal.

5. Appareil de stimulation (1,21) selon la revendication 3 ou 4, **caractérisé en ce que** l'entrée de commande de l'élément de commutation commandable (11,40) est reliée, pour le branchement et le débranchement, commandés dans le temps, du capteur hémodynamique (4,38 à 41), à une minuterie (28,42) ou, pour le branchement et le débranchement télécommandés, par un appareil de contrôle extracorporel à un récepteur de télémétrie.

6. Appareil de stimulation (1,21) selon la revendication 5, **caractérisé en ce que** l'unité de commande (2) comporte, pour l'évaluation aussi bien du débit sanguin mesuré que des signaux cardiaques, une unité logique (7), qui est reliée côté entrée pour l'évaluation des signaux cardiaques à l'unité (6) d'analyse des signaux cardiaques et pour l'évaluation de la puissance de pompage du coeur, à l'unité (14) d'analyse des signaux de débit sanguin.

7. Appareil de stimulation (1,21) selon la revendication 1, **caractérisé en ce que** les deux électrodes de mesure (51.1,51.2) sont disposées dans le vaisseau sanguin (50) l'un derrière l'autre pour la mesure d'impédance longitudinale dans la direction d'écoulement ou l'un à côté de l'autre pour la mesure d'impédance, transversalement par rapport à la direction d'écoulement.

8. Appareil de stimulation (1,21) selon l'une des revendications précédentes, **caractérisé en ce que** le capteur hémodynamique (4,45) est disposé dans la veine cave (37) ou dans l'artère pulmonaire.

9. Appareil de stimulation selon l'une des revendications 1 à 8, **caractérisé en ce qu'**une tension de mesure située dans la gamme de 0,3 V à 3 V, notamment égale à environ 1 V, et un courant de mesure situé dans la gamme de 10 µA à 100 µA sont réglés par le générateur de signaux (53) et la résistance série (53a).

10. Appareil de stimulation selon l'une des revendications précédentes, **caractérisé en ce que** des moyens (43a) sont prévus pour compenser l'influence de variations du signal de débit sanguin conditionnées par la température et/ou par une médication.
